(19) Europäisches Patentamt
European Patent Office
Office européen des brevets

(11) Veröffentlichungsnummer : **0 099 056**
**B1**

(12) **EUROPÄISCHE PATENTSCHRIFT**

(45) Veröffentlichungstag der Patentschrift : **30.09.87**

(51) Int. Cl.⁴ : **C 07 K 1/00**, C 07 K 5/06, C 07 K 5/08, A 61 K 37/02

(21) Anmeldenummer : **83106586.7**

(22) Anmeldetag : **06.07.83**

(54) **N-Acylderivate von Dipeptiden, ihre Herstellung und Verwendung bei der Therapie von Krankheiten und Mittel dafür.**

(30) Priorität : **14.07.82 DE 3226242**

(43) Veröffentlichungstag der Anmeldung : **25.01.84 Patentblatt 84/04**

(45) Bekanntmachung des Hinweises auf die Patenterteilung : **30.09.87 Patentblatt 87/40**

(84) Benannte Vertragsstaaten : **AT BE CH DE FR GB IT LI NL SE**

(56) Entgegenhaltungen :
**US-A- 3 778 428**
**COLLECTION OF CZECHOSLOVAK CHEMICAL COMMUNICATIONS, Band 45, 1980, Seiten 294-297, Academia, Prague, CZ; E. KASAFIREK et al.: "Synthesis of analogues of the melanocyte-stimulating hormone release-inhibiting factor containing 2-oxoimidazolidine-1-carboxylic acid"**
**CHEMICAL ABSTRACTS, Band 95, 1981, Seite 767, Nr. 62675u, Columbus, Ohio, US; K. BREDDAM et al.: "Influence of the substrate structure on carboxypeptidase Y catalyzed peptide bond formation"**
**JOURNAL OF MEDICINAL CHEMISTRY, Band 22, Nr. 8, August 1979, Seiten 931-933, American Chemical Society, US; S. BJÖRKMAN et al.: "Tripeptide analogues of melanocyte-stimulating hormone release-inhibiting hormone (Pro-Leu-Gly-NH2) as inhibitors of oxotremorine-induced tremor"**
**CHEMICAL ABSTRACTS, Band 55, Nr. 20, 2. Oktober 1961, Spalte 20034d, Columbus, Ohio, US; S. AONUMA et al.: "Posterior pituitary hormones. III. Inactivation of oxytocin."**
**CHEMICAL ABSTRACTS, Band 95, 1981, Seite 766, Nr. 62664q, Columbus, Ohio, US; A.M. EL-NAGGAR et al.: "Synthesis of some 2-hydroxybenzoyl di- and tripeptide derivatives"**

(73) Patentinhaber : **BASF Aktiengesellschaft**
**Carl-Bosch-Strasse 38**
**D-6700 Ludwigshafen (DE)**

(72) Erfinder : **Albrecht, Hans Peter, Dr.**
**Brombeerweg 5**
**D-6940 Weinheim (DE)**
Erfinder : **Kreiskott, Horst, Dr.**
**Am Boehlig**
**D-6706 Wachenheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 099 056 B1

Jouve, 18, rue St-Denis, 75001 Paris, France

## Beschreibung

Die Erfindung betrifft neue N-Acylderivate von Dipeptiden, Verfahren zu deren Herstellung, Arzneimittel, die diese neuen Verbindungen enthalten, sowie deren Verwendung zur Therapie von Krankheiten.

Das Tripeptid L—Pro—L—Leu—Gly—$NH_2$ (MIF) ist der Hemmfaktor des Melanocyten-stimulierenden Hormons (The Merck-Index, 9. Aufl. 1976). Neben seiner endokrinen Wirkung übt das Tripeptid im Zentralnervensystem einen Neurotransmitter- oder Neuromodulator-Effekt aus. Klinische Studien zeigten, daß MIF allein oder in Kombination mit L-Dopa bei Parkinson-Patienten Tremor, Rigor und Akinese günstig beeinflußt (A. J. Kastin u. A. Barbeau Can. Med. Assoc. J. 107, 1097 (1972) und F. Gerstenbrand et al., Wien. klin. Wschr. 87, 822 (1975)).

Einer breiten therapeutischen Anwendung dieses Tripeptids steht jedoch die ungenügende orale Wirksamkeit und die kurze Wirkdauer im Wege.

Es ist weiter versucht worden — unter Erhaltung der pharmakologischen Wirksamkeit von L—Pro—L—Leu—Gly—$NH_2$ — durch Molekülabwandlung zu Verbindungen mit oraler Wirksamkeit und ausreichender Wirkdauer zu kommen.

Die Bemühungen konzentrierten sich auf den Austausch des mittelständigen L-Leucins gegen D-Leucin (US-PS 4 278 595) oder gegen N-Alkylderivate von L- und D-Leucin (DE-OS 26 33 976).

Der Ersatz des L-Prolins durch Säuren oder Aminosäuren wurde bisher nur wenig untersucht, da er, abgesehen vom Austausch gegen L-Pyroglutaminsäure, der unschädlich ist (S. Björkman et al. Acta. Pharm. Suec. 13, 289 (1976)), nur zu pharmakologisch inaktiven Produkten geführt hat (R. C. Johnson et al., J. Med. Chem. 21, 165 (1978)).

Weiter sind in Coll. Czech., Chem. Communic. 45, 294 (1980) geringfügig modifizierte MIF-Analoge beschrieben. Durch diese Veränderungen sind jedoch keine Wirkungssteigerungen erzielt worden. Schließlich ist es aus J. Med. Chem. 21, (1978) bekannt, daß (fast) isostere Änderungen im Molekül — wie der Austausch von Pyrrolidin durch Cyclopentan oder Thiazolidin — zu einem Wirkungsverlust oder Wirkungsabfall führen.

Es wurde nun gefunden, daß N-Acylderivate von Dipeptiden der Formel I

$$X—CO—L—Leu—NH—CH_2—CO—R \qquad I,$$

in der

X einen gesättigten cycloaliphatischen Kohlenwasserstoffrest mit 3 bis 10 Kohlenstoffatomen oder einen Indanyl- oder 1,2,3,4-Tetrahydronaphthalinrest bedeutet, in welchen Resten das C-Atom, welches mit der Carbonylgruppe verbunden ist, noch eine Aminogruppe tragen kann, einen L-Homopyrrolidinylrest oder ein über ein C-Atom gebundenes ein gesättigtes oder ungesättigtes 4- bis 6-gliedriges heterocyclisches Ringsystem mit bis zu 2 Heteroatomen darstellt, wobei der Heterocyclus auch mit einem Benzolkern kondensiert sein kann und wobei — falls der Heterocyclus Stickstoff enthält — dieser auch eine Oxo- oder eine $C_1$-$C_3$-Alkylgruppe enthalten kann und

R eine $C_1$-$C_5$-Alkoxy- oder der Rest $NR^1R^2$ ist, worin $R^1$ und $R^2$ gleich oder verschieden sind und Wasserstoffatome oder $C_1$-$C_5$-Alkylgruppen bedeuten,
wobei jedoch X

a) kein Pyrrolidin-2-ylrest oder 5-Oxo-pyrrolidin-2-ylrest
b) kein Cyclopentylrest, wenn R eine Amino- oder Ethylaminogruppe bedeutet, und
c) kein 4-Thiazolidinrest, wenn R eine Aminogruppe bedeutet,

sein kann, sowie deren Salze mit physiologisch verträglichen Säuren allein oder in Kombination mit L-Dopa gute Therapeutika sind.

In der Formel I bedeutet X vorzugsweise einen gesättigten cycloaliphatischen Kohlenwasserstoffrest mit 5 bis 7 Kohlenstoffatomen oder einen Indanyl- oder 1,2,3,4-Tetrahydronaphthalinrest, in welchen Resten das C-Atom, welches mit der Carbonylgruppe verbunden ist, noch eine Aminogruppe trägt. Weiter sind die Verbindungen bevorzugt, in denen X ein gesättigtes oder ungesättigtes Ringsystem mit 4 bis 5 C-Atomen und einem N-Atom darstellt, worin ein C-Atom durch ein S-Atom oder ein weiteres N-Atom ersetzt sein kann und wobei der Heterocyclus mit einem Benzolkern kondensiert ist und an einem dem N-Atom benachbarten C-Atom gegebenenfalls eine Oxogruppe trägt. Der Rest —CO—L—Leu—NH—$CH_2$—CO—R steht in diesen Verbindungen an einem C-Atom, welches dem N-Atom benachbart ist.

Besonders bevorzugt sind diejenigen Verbindungen, in denen X die oben genannten Bedeutungen besitzt und R den Rest $NR^1R^2$ bedeutet.

Als physiologisch verträgliche Säuren kommen insbesondere in Betracht : Salzsäure, Schwefelsäure, Phosphorsäure, Essigsäure, Milchsäure, Bernsteinsäure, Fumarsäure und Äpfelsäure.

Die neuen N-Acyl-dipeptide der Formel I lassen sich nach Methoden darstellen, wie sie allgemein in der Peptid-Chemie üblich sind. Die verschiedenen Reaktionen werden nach Blockierung der Aminfunktion durch geeignete Schutzgruppen, die in die Reaktion nicht eingreifen, durchgeführt. Die Abspaltung

der Schutzgruppen nach erfolgter Reaktion geschieht ebenfalls nach dem üblichen Verfahren der Peptid-Chemie (vgl. E. Wünsch in « Methoden der Organischen Chemie » Band XV/1 und XV/2, Herausgeber E. Müller, Georg Thieme Verlag Stuttgart 1974).

Die Herstellung der neuen Verbindungen erfolgt insbesondere dadurch, daß man

a) falls X keine basische Aminogruppe enthält — eine Carbonsäure der Formel II

$$X—COOH \qquad II,$$

auf ein Dipeptid der Formel III

$$L—Leu—NH—CH_2—CO—R \qquad III,$$

worin R die angegebene Bedeutung besitzt, einwirken läßt oder

b) falls X eine basische Aminogruppe enthält — die an der Aminogruppe durch eine Schutzgruppe geschützte Verbindung der Formel II mit dem Dipeptid der Formel III kondensiert und anschließend die Schutzgruppe abspaltet

und die so erhaltenen Verbindungen gegebenenfalls in ihre Salze mit physiologisch verträglichen Substanzen überführt.

Carbonsäuren der allgemeinen Formel I bzw. deren aktivierte Derivate, die ein asymmetrisch substituiertes Kohlenstoffatom besitzen, können entweder in Form ihrer optisch reinen Formen oder in Form ihres Racemats eingesetzt werden. Im letzteren Fall entstehen bei der Kondensation mit dem optisch aktiven Dipeptid der Formel II Diastereomeren-Gemische, die sich chromatographisch oder durch Kristallisation trennen lassen.

Für die Umsetzung a) ist es im allgemeinen notwendig, die freie Säurefunktion der Carbonsäure der Formel II vor ihrer Einwirkung auf das Dipeptid der Formel III zu aktivieren. Aktivierte Derivate der Carbonsäure sind bevorzugt die gemischten Anhydride, die in situ durch Einwirkung eines Chlorameisensäurealkylesters — wie beispielsweise Chlorameisensäureisobutylester oder Chlorameisensäureethylester — dargestellt werden, Additionsprodukte an Carbodiimide — vorzugsweise Dicyclohexylcarbodiimid-oder aktivierte Ester, vorzugsweise die aus N-Hydroxysuccinimid und Dicyclohexylcarbodiimid gegebenenfalls in situ herstellbaren N-Hydroxysuccinimidester. Die Kondensation des aktivierten Derivats erfolgt in einem organischen Lösungsmittel wie Dioxan, Tetrahydrofuran, Dichlormethan, Chloroform, Toluol oder Dimethylformamid oder in einem wäßrigorganischen Medium in Gegenwart einer Base. Als Basen werden vorzugsweise Triethylamin, N-Methylmorpholin oder Natriumhydrogencarbonat verwendet. Die Reaktionstemperatur liegt zwischen — 10 bis + 30 °C, die Reaktionszeit beträgt 3 Stunden bis 4 Tage.

Für das Verfahren b) verwendet man die in der Peptid-Chemie gebräuchlichen Aminoschutzgruppen. Bevorzugt sind Benzyloxycarbonyl, t-Butyloxycarbonyl oder Benzhydryl.

Im allgemeinen ist es notwendig, die freie Säurefunktion der Carbonsäure vor ihrer Einwirkung auf das Dipeptid der Formel III zu aktivieren. Aktivierte Derivate der Carbonsäure sind bevorzugt die gemischten Anhydride, die in situ durch Einwirkung eines Chlorameisensäurealkylesters — wie beispielsweise Chlorameisensäureisobutylester oder Chlorameisensäureethylester — hergestellt werden, Additionsprodukte an Carbodiimide — vorzugsweise Dicyclohexylcarbodiimid — oder aktivierte Ester, vorzugsweise die Aus N-Hydroxysuccinimid und Dicyclohexylcarbodiimid gegebenenfalls in situ herstellbaren N-Hydroxysuccinimidester.

Die Kondensation des aktivierten Derivats erfolgt in einem organischen Lösungsmittel wie Dioxan, Tetrahydrofuran, Dichlormethan, Chloroform, Toluol oder Dimethylformamid oder in einem wäßrigorganischen Medium in Gegenwart einer Base. Als Base werden vorzugsweise Triethylamin, N-Methylmorpholin oder Natriumhydrogencarbonat verwendet. Die Reaktionstemperatur liegt zwischen — 10 und + 30 °C, die Reaktionszeit beträgt 3 Stunden bis 4 Tage.

Bei der Kondensationsreaktion entstehen geschützte Derivate der Verbindungen der Formel I. Diese werden durch Schutzgruppenabspaltung in die erfindungsgemäße Verbindung der Formel I überführt. Ist die Schutzgruppe Benzyloxycarbonyl oder Benzhydryl, so erfolgt die Abspaltung zweckmäßig durch Hydrierung in Gegenwart eines Edelmetallkatalysators in einem inerten Lösungsmittel bei Raumtemperatur. Bevorzugte Edelmetallkatalysatoren sind Palladium, Platin oder Raney-Nickel. In der bevorzugten Ausführungsform kommt 10 %iges Palladium auf Kohle zur Anwendung. Als Lösungsmittel werden vorzugsweise Methanol Essigsäureethylester oder Eisessig verwendet. Ist die Schutzgruppe t-Butyloxycarbonyl, erfolgt die Abspaltung zweckmäßig durch Behandeln des Zwischenprodukts mit einem Überschuß von Trifluoressigsäure oder mit einer Lösung von Chlorwasserstoff in einem inerten organischen Lösungsmittel wie Essigsäureethylester, Dioxan oder Tetrahydrofuran. Die Abspaltungsreaktion wird bei 0 bis 20 °C durchgeführt. Die Reaktionsdauer beträgt 5 bis 30 min.

Die neuen N-Acylderivate von Dipeptiden sind gegenüber der Einwirkung proteolytischer Enzyme weitgehend stabil, nach oraler Gabe wirksam und besitzen eine lange Wirkdauer.

Die Überlegenheit der neuen Substanzen zeigt sich insbesondere in folgenden Testmodellen :

1. Nach Everett, G. M. (in : Antidepressant Drugs, eds. Garattini, S. and Dukes, M. N. G, Amsterdam

1967, p. 164 ff.) wird eine Kombination von L-Dopa und Pargylin, die ein schwaches Erregungsbild hervorruft, Mäusen verabfolgt. Durch Vorbehandlung mit zentral erregenden Substanzen entwickelt sich erst ein ausgeprägtes Erregungsbild. Die neuen Tripeptide sind in diesem Testmodell bei oraler Verabfolgung in Dosierungen ab 0,02 mg/kg wirksam.

2. Die Applikationen des Cholinomimetikums Pilokarpin an der Ratte ruft neben peripheren Symptomen zentral Kratzen hervor (Kreiskott, H., Arch. exp. Path. Pharmak. 247, 317 (1964), das sich durch zentrale Anticholinergica, aber auch durch zentrale monaminerge Stimulantien (Kreiskott, H. and Hofmann, H. P., 6. Int. Congress Pharmacol., Helsinki 1975, Abstr. 825) verhindern läßt. Orale Vorbehandlung mit den erfindungsgemäßen Tripeptiden unterdrückt das Pilokarpin-induzierte zentrale Kratzen.

3. Die in den Testmodellen 1 und 2 wirksamen Substanzen wurden zusätzlich an der Maus in einem Wirkungs- und Vergiftungsbild geprüft. Dabei werden die Symptome nach dem Verfahren von S. Irwin [Psychopharmakologia 13, 222 (1968)] erfaßt und quantifiziert. Die verschiedenen Testparameter werden kurz vor Substanzgabe sowie 1/2, 1, 2, 3 und 24 Stunden nach oraler Gabe, gemessen. Je Dosis werden Gruppen von 3 Tieren verwendet, die erste Verhaltensprüfung erfolgt nach 30 Minuten Eingewöhnung an den Makrolon-Käfig. Erfaßt werden Grundverhalten, zentrale Stimulation und Dämpfung sowie vegetative Symptome. Im einzelnen sind dies :

Körperhaltung
Stellung der Gliedmaßen
Putzverhalten
Stupor
spontane und induzierte lokomotorische Aktivität
Atmung
sensomotorische Reaktionen (Reflexe)
Lidspaltenweite
Pupillengröße und
Körpertemperatur u. a.

die neuen Substanzen rufen eine Steigerung der Lokomotorik sowie verstärktes Schnüffeln, Aufrichten und Putzen hervor. Diese Symptomatik tritt gleichermaßen bei dopaminerg wirksamen und Dopaminstimulierenden Substanzen auf.

Die neuen Substanzen stimulieren demnach dopaminerge Abläufe deutlich. Im Testmodell 1 (L-Dopa-Potenzierung) wird die Wirkung exogen zugeführten Dopamins gesteigert, in 2 (Pilokarpinerregung) und 3 (Wirkungsbild) wird das endogen vorhandene Dopamin in seinem Effekt verstärkte.

4. Mit mittleren Dosen Morphin subkutan vorbehandelte Ratten sind vom Gesamtverhalten her nicht auffällig. Kommt jedoch ein zusätzlicher äußerer Reiz hinzu, wie das Aufsetzen einer Klammer an den Schwanz, erstarren die Tiere schlagartig und zeigen Katalepsie. Nach Wegfall des Reizes sind die Ratten wieder unauffällig (Stille, G. Zur Pharmakologie katatonigener Stoffe, Aulendorf 1971 p. 30). Dieser reizinduzierte Zustand läßt sich durch intravenöse Injektion mit den beanspruchten Tripeptiden verhindern.

Die erfindungsgemäßen Peptide eignen sich daher allein oder in Kombination mit L-Dopa zur oralen Therapie des Morbus Parkinson und parkinsonoider Zustände sowie von Depressionen. Ferner können sie zur Verhinderung oder Behandlung der Opiatabhängigkeit eingesetzt werden.

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen etwa 0,1 und 100 mg/kg Körpergewicht bei oraler Gabe und zwischen etwa 0,01 und 10 mg/kg Körpergewicht bei parenteraler Gabe.

Die neuen Verbindungen können in den gebräuchlichen galenischen Applikationsformen fest oder flüssig abgewendet werden, z. B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al : Pharmazeutische Technologie, Thieme-Verlag, Stuttgart, 1978). Die so erhaltenen Applikationsformen enthalten den Wirkstoff normalerweise in einer Menge von 0,1 bis 99 Gew.%.

Experimenteller Teil

Die folgenden Beispiele dienen zur Erläuterung der Erfindung, ohne diese zu beschränken.

Der Verlauf aller Reaktionen wurde mittels Dünnschichtchromatographie auf Kieselgelfertigplatten $F_{254}$ (Fa. Merck) verfolgt. Als Fließmittel wurden abhängig von der Polarität der untersuchten Verbindungen Dichlormethan/Aceton 20 : 1 bis 5 : 1, Dichlormethan/Methanol 20 : 1 bis 2 : 1 oder Butanol/Essigsäureethylester/Eisessig/Wasser 4 : 1 : 1 : 1 verwendet.

Die nach den folgenden Beispielen erhaltenen neuen Verbindungen der allgemeinen Formel I sind dünnschichtchromatographisch rein.

Die NMR-Spektren sind im Einklang mit der angegebenen Struktur.

Celit(R) ist ein Filtrierhilfsmittel der Firma Johns-Manville.

Beispiel 1

Pyrrol-2-yl-carbonyl-L-leucyl-glycinamid

Zu 9,4 g L-Leucyl-glycinamid in 120 ml Dioxan wurden bei 10 °C 7,0 ml Triethylamin und 10,4 g des N-Hydroxysuccinimidesters der Pyrrol-2-carbonsäure zugefügt. Das Reaktionsgemisch wurde 2 h bei 10 °C gerührt, anschließend wurden 30 ml Wasser zugegeben und 20 h bei Raumtemperatur unter Rühren umgesetzt.

Zur Aufarbeitung wurde in Essigsäureethylester aufgenommen und die organische Phase nacheinander mit 10 %iger wäßriger Zitronensäure-Lösung, Soda-Lösung und Wasser gewaschen, über Natriumsulfat getrocknet und im Vakuum eingedampft. Chromatographie des Rückstandes an einer Kieselgelsäule (Elution mit Dichlormethan/Methanol 10 : 1) gab 5,0 g (36 %) Pyrrol-2-yl-carbonyl-L-leucyl-glycinamid, $[\alpha]_D^{20}$ = — 2° (c = 0,5 Methanol).

Analog Beispiel 1 wurden folgende Verbindungen erhalten :

2. Thiophen-2-yl-carbonyl-L-leucyl-glycinamid (56 %), Fp = 155 bis 165 °C (Dichlormethan/Ether/-Petrolether), $[\alpha]_D^{20}$ = — 11° (c = 0,5 Methanol) ;
3. Furan-2-yl-carbonyl-L-leucyl-glycinamid (44 %), $[\alpha]_D^{20}$ = + 5° (c = 0,5 Methanol) ;
4. Thiophen-3-yl-carbonyl-L-leucyl-glycinamid (46 %), $[\alpha]_D^{20}$ = — 3° (c = 0,3 Methanol) ;
5. D,L-Tetrahydrofuran-2-yl-carbonyl-L-leucyl-glycinamid (56 %), Fp = 109 bis 122 °C (Dichlormethan/Hexan), $[\alpha]_D^{20}$ = — 8° (c = 0,5 Methanol) ;
6. D,L-Tetrahydrothiophen-2-yl-carbonyl-L-leucyl-glycinamid (32 %), Fp = 149 bis 151 °C (Dichlormethan/Ether), $[\alpha]_D^{20}$ = — 12° (c = 0,5 Methanol) ;
7. Cyclobutylcarbonyl-L-leucyl-glycinamid (41 %), Fp = 126 bis 136 °C (Dichlormethan/Ether), $[\alpha]_D^{20}$ = — 20° (c = 0,5 Methanol) ;
8. Cyclohexylcarbonyl-L-leucyl-glycinamid (54 %), Fp = 196 bis 197 °C (Dichlormethan/Methanol/Ether) ;
9. Indol-2-yl-carbonyl-L-leucyl-glycinamid (58 %), Fp = 203 bis 204 °C (Dichlormethan/Methanol/Ether), $[\alpha]_D^{20}$ = 8° (c = 0,5 Methanol) ;
10. Benzoyl-L-leucyl-glycinamid (55 %), Fp = 147 bis 150 °C (Dichlormethan/Ether) ;
11. Pyrid-3-yl-carbonyl-L-leucyl-glycinamid (38 %), $[\alpha]_D^{20}$ = + 4° (c = 0,5 Methanol) ;
12. Indol-2-yl-carbonyl-L-leucyl-glycinethylester (63 %), $[\alpha]_D^{20}$ = — 9° (c = 0,5 Methanol) ;
13. Pyrrol-2-yl-carbonyl-L-leucyl-glycinethylester (55 %), Fp = 169 bis 170 °C (Methanol/Ether), $[\alpha]_D^{20}$ = — 25° (c = 0,5 Methanol) ;
14. L-Piperid-2-on-6-yl-carbonyl-L-leucyl-glycinamid (31 %), Fp = 98 bis 108 °C, $[\alpha]_D^{20}$ = — 4° (c = 0,5 Methanol).

Beispiel 15

Adamant-1-yl-carbonyl-L-leucyl-glycinamid

Zu 2,7 g Adamantan-1-carbonsäure und 2,0 ml Triethylamin in 40 ml Dimethylformamid wurden bei — 10 °C unter Rühren 2,0 ml Chlorameisensäureisobutylester zugetropft. Nach 15 min wurden zu der erhaltenen Lösung des asymmetrischen Säureanhydrids 2,8 g L-leucyl-glycinamid zugegeben. Man rührte 1 h bei — 10 °C und anschließend 16 h bei Raumtemperatur und dampfte im Vakuum ein.

Zur Aufarbeitung wurde in Essigsäureethylester aufgenommen und nacheinander mit 10 %iger wäßriger Zitronensäure-Lösung, Soda-Lösung und Wasser gewaschen, über Natriumsulfat getrocknet und im Vakuum eingedampft. Chromatographie des Rückstands an einer Kieselgelsäule (Elution mit Dichlormethan/Methanol 10 : 1) gab 2,7 g (52 %) Adamant-1-yl-carbonyl-L-leucyl-glycinamid, Fp = 123 bis 127 °C (Dichlormethan/Ether/Hexan).

Analog Beispiel 15 wurde erhalten :

16. Pyrid-2-yl-carbonyl-L-leucyl-glycinamid (52 %), Fp = 141 bis 146 °C (Dichlormethan/Hexan), $[\alpha]_D^{20}$ = + 7° (c = 0,5 Methanol).

Beispiel 17

D-1,2,3,4-Tetrahydrochinol-2-yl-carbonyl-L-leucyl-glycinamid

und
L-1,2,3,4-Tetrahydrochinol-2-yl-carbonyl-L-leucyl-glycinamid

Zu 5,6 g L-Leucyl-glycinamid in 60 ml Dioxan wurden bei 10 °C 8,4 ml Triethylamin und 12,3 g des N-Hydroxysuccinimidesters von 1,2,3,4-Tetrahydrochinolin-2-carbonsäure zugefügt. Das Reaktionsgemisch wurde 3 h bei 10 °C gerührt, anschließend wurden 30 ml Wasser zugegeben und 20 h bei Raumtemperatur umgesetzt. Es wurde in Essigsäureethylester aufgenommen, die organische Phase nacheinander mit 10 %iger wäßriger Zitronensäure-Lösung, Soda-Lösung und Wasser gewaschen, über Natriumsulfat getrocknet und im Vakuum eingedampft. Durch Chromatographie an einer Kieselgelsäule und Elution mit Dichlormethan/Methanol 20 : 1 erhielt man die beiden Isomeren in reiner Form :

Benzyloxycarbonyl-D-1,2,3,4-tetrahydrochinol-2-yl-carbonyl-L-leucyl-glycinamid, Ausbeute 3,8 g (26 %), Fp = 163 bis 173 °C (Isopropanol/Ether), $[\alpha]_D^{20}$ = + 23° (c = 0,5 Methanol) ;

Benzyloxycarbonyl-L-1,2,3,4-tetrahydrochinol-2-yl-carbonyl-L-leucyl-glycinamid, Ausbeute 2,9 g (20 %), Fp = 142 bis 145 °C (Isopropanol/Diisopropylether, $[\alpha]_D^{20}$ = — 88° (c = 0,5 Methanol).

2,1 g Benzyloxycarbonyl-D-1,2,3,4-tetrahydrochinol-2-L-leucyl-glycinamid werden in 150 ml Methanol gelöst und in Gegenwart von 0,2 g Palladium auf Kohle (10 %) hydriert. Nach Aufnahme der theoretischen Menge Wasserstoff wurde über Celit filtriert, das Filtrat im Vakuum eingedampft und der Rückstand aus Essigsäureethylester/Ether/Hexan kristallisiert. Ausbeute : 1,0 g (66 %) an D-1,2,3,4-Tetrahydrochinol-2-yl-carbonyl-L-leucyl-glycinamid, Fp = 137 bis 140 °C, $[\alpha]_D^{20}$ = + 36° (c = 0,5 Methanol).

Analog wurde aus Benzyloxycarbonyl-L-1,2,3,4-Tetrahydrochinol-2-yl-carbonyl-L-leucyl-glycinamid 1,5 g (70 %) L-1,2,3,4-Tetrahydrochinol-2-yl-carbonyl-L-leucyl-glycinamid erhalten, Fp = 73 bis 85 °C (Essigsäureethylester/Ether/Petrolether, $[\alpha]_D^{20}$ = — 50° (c = 0,5 Methanol).

## Beispiel 18

Analog Beispiel 17 aber mit der Änderung, daß die chromatographische Trennung der beiden Isomeren an einer Kieselgelsäule (Elution mit Dichlorethan/Methanol 10 : 1) erst auf der Stufe des Endproduktes erfolgt, erhielt man in der Reihenfolge steigender Polarität :

18a) L-1,2,3,4-Tetrahydroisochinol-3-yl-carbonyl-L-leucyl-glycinamid, Ausbeute 40 % d. Theorie, Fp. = 160 bis 163 °C, $[\alpha]_D^{20}$ = — 69° (c = 0,5 Methanol) und

18b) D-1,2,3,4-Tetrahydroisochinol-3-yl-carbonyl-L-leucyl-glycinamid, Ausbeute 20 % d. Theorie, Fp. = 176 bis 181 °C, $[\alpha]_D^{20}$ = + 42° (c = 0,5 Methanol).

## Beispiel 19

4,5-Dehydro-piperid-2-yl-carbonyl-L-leucyl-glycinamid

Zu 9,1 g N-tert.-Butyloxycarbonyl-4,5-dehydro-piperidin-2-carbonsäure und 6,1 ml Triethylamin in 100 ml absolutem Tetrahydrofuran wurden bei — 10 °C unter Rühren 6,0 ml Chlorameisensäureisobutylester zugetropft. Nach 15 min wurden zu der so erhaltenen Lösung des asymmetrischen Säureanhydrids 2,2 g L-leucyl-glycinamid in 100 ml Dimethylformamid/Tetrahydrofuran (1 : 2) gegeben. Man rührte 1 h bei — 10 °C, dann 16 h bei Raumtemperatur, anschließend wurde im Vakuum eingedampft. Zur Aufarbeitung wurde in Essigsäureethylester aufgenommen und nacheinander mit 10 %iger wäßriger Zitronensäure-Lösung, Soda-Lösung und Wasser gewaschen, über Natriumsulfat getrocknet und im Vakuum eingedampft. Reinigung an Kieselgel (Elution mit Dichlormethan/Methanol 0 : 1) gab 9,0 g (57 %) N-tert.-Butyloxycarbonyl-4,5-dehydro-piperid-2-yl-carbonyl-L-leucyl-glycinamid.

Dieses wurde 15 min bei Raumtemperatur mit 100 ml einer ca. 6 N Lösung von Chlorwasserstoff in Dioxan behandelt. Man dampfte anschließend im Vakuum ein. Der Rückstand wurde mehrfach mit Toluol zusammen evaporiert und aus Methanol/Ether kristallisiert.

Ausbeute 5,6 g (74 % 4,5-Dehydro-piperid-2-yl-carbonyl-L-leucyl-glycinamid-Hydrochlorid, Fp = 213 bis 215 °C (Methanol/Ether), $[\alpha]_D^{20}$ = — 23° (c = 0,5 Methanol).

## Beispiel 20

L-Homopropyl-L-leucyl-glycinamid

Zu 4,2 g L-Leucyl-glycinamid in 30 ml Dioxan wurden bei 10 °C 6,3 ml Triethylamin und 11,5 g des N-Hydroxysuccinimidesters von N-Benzyloxycarbonyl-L-homoprolin zugefügt. Das Reaktionsgemisch wurde 2 h bei 10 °C gerührt, anschließend wurden 5 ml Wasser zugegeben und 20 h bei Raumtemperatur umgesetzt. Zur Aufarbeitung wurde in Essigsäureethylester aufgenommen und nacheinander mit 10 %iger wäßriger Zitronensäure-Lösung, Soda-Lösung und Wasser gewaschen, über Natriumsulfat getrocknet und im Vakuum eingedampft. Der Rückstand wurde durch Chromatographie an einer

Kieselgelsäure gereinigt (Elution mit Dichlormethan/Methanol 15 : 1). Man erhielt 1,8 g N-Benzyloxycarbonyl-L-homoprolyl-L-leucyl-glycinamid. Dieses wurde in 100 ml Methanol gelöst und in Gegenwart von 0,3 g Palladium auf Kohle (10 %) hydriert. Nach Filtration über Celit(R) wurde im Vakuum eingedampft. Ausbeute 0,8 g (90 %) L-Homoprolyl-L-leucyl-glycinamid, $[\alpha]_D^{20}$ = — 13,6° (c = 0,5 Methanol).

Analog Beispiel 20 wurden folgende Verbindungen erhalten :

21. D,L-Piperid-2-yl-carbonyl-L-leucyl-glycinamid (35 %), Fp = 75 bis 80 °C (Essigsäureethylester/Ether), $[\alpha]_D^{20}$ = — 16° (c = 0,5 Methanol).
22. Anthranyl-L-leucyl-glycinamid (55 %), Fp = 64 bis 69 °C, $[\alpha]_D^{20}$ = — 37° (c = 0,5 Methanol).
23. L-3-Methyl-prolyl-L-leucyl-glycinamid (43 %), Fp = 110 bis 116 °C (Methanol/Isopropylether).

## Beispiel 24

L-Azetidin-2-yl-carbonyl-L-leucyl-glycinamid und
D-Azetidin-2-yl-carbonyl-L-leucyl-glyinamid

Zu 1,9 g L-leucyl-glycinamid in 10 ml Dioxan wurden bei 10 °C 2,6 ml Triethylamin und 3,6 g des N-Hydroxysuccinimidesters von Benzhydrylazetidin-2-carbonsäure zugegeben. Nach 2 h bei 10 °C wurden 5 ml Wasser zugegeben und das Reaktionsgemisch 20 h bei Raumtemperatur gerührt. Zur Aufarbeitung wurde in Essigsäureethylester aufgenommen, und nacheinander mit 10 %iger wäßriger Zitronensäure-Lösung, Soda-Lösung und Wasser gewaschen, über Natriumsulfat getrocknet und im Vakuum eingedampft. Durch Chromatographie an einer Kieselgelsäule (Elution mit Dichlormethan/Methanol 15 : 1) wurden in der Reihenfolge steigender Polarität die beiden Isomeren in reiner Form erhalten :

1,3 g (30 %) Benzhydryl-L-azetidin-2-yl-carbonyl-L-leucyl-glycinamid, Fp = 88 bis 90 °C ;
1,7 g (39 %) Benzhydryl-D-azetidin-2-yl-carbonyl-L-leucyl-glycinamid, Fp = 90 bis 95 °C.

1,3 g Benzhydryl-L-azetidin-2-yl-carbonyl-L-leucyl-glycinamid wurden in Methanol gelöst und in Gegenwart von 0,3 g Palladium auf Kohle (10 %) hydriert. Nach Filtration über Celit(R) und Eindampfen im Vakuum wurden 0,75 g (94 %) L-Azetidin-2-yl-carbonyl-L-leucyl-glycin-amid erhalten, $[\alpha]_D^{20}$ = — 75° (c = 0,5 Methanol).

Analog wurden aus Benzhydryl-D-azetidin-2-yl-carbonyl-L-leucyl-glycinamid 1,5 g (90 %) D-Azetidin-2-yl-carbonyl-L-leucyl-glycinamid erhalten, Fp = 110 bis 120 °C, $[\alpha]_D^{20}$ = + 16° (c = 0,5 Methanol).

## Beispiel 25

D-2-Methylprolyl-L-leucyl-glycinamid und L-2-Methylprolyl-L-leucyl-glycinamid

Zu 13,0 g Benzyloxycarbonyl-D,L-2-methylprolin und 7 ml Triethylamin in 100 ml Tetrahydrofuran wurden bei — 10 °C unter Rühren 7 ml Chlorameisensäureisobutylester zugetropft. Nach 15 min wurden zu der erhaltenen Lösung des asymmetrischen Säureanhydrids 9,5 g L-Leucyl-glycinamid in 50 ml Dimethylformamid zugegeben. Nach 1 h bei — 10 °C wurde 24 h bei Raumtemperatur weitergerührt. Zur Aufarbeitung wurde in Essigsäureethylester aufgenommen und nacheinander mit 10 %iger Zitronensäure-Lösung, Soda-Lösung und Wasser gewaschen und anschließend im Vakuum eingedampft. Chromatographie des Rückstands an einer Kieselgelsäule (Elution mit Toluol/Methanol 15 : 1) gab in der Reihenfolge steigender Polarität die beiden Isomeren in reiner Form :

3,7 g (17 %) Benzyloxycarbonyl-D-2-methylprolyl-L-leucyl-glycinamid
und
33 g (15 %) Benzyloxycarbonyl-L-2-methylprolyl-L-leucyl-glycinamid.

0,9 g Benzyloxycarbonyl-D-2-methylprolyl-L-leucyl-glycinamid wurden in Methanol in Gegenwart von 0,2 g Palladium auf Kohle (10 %) hydriert. Nach Filtration über Celit(R) und Eindampfen im Vakuum wurden 0,6 g (97 %) D-2-Methylprolyl-L-leucyl-glycinamid erhalten, $[\alpha]_D^{20}$ = — 38° (c = 0,5 Methanol).

Analog erhielt man aus Benzyloxycarbonyl-L-2-methylprolyl-L-leucyl-glycinamid, L-2-Methylprolyl-L-leucyl-glycinamid $[\alpha]_D^{20}$ = + 11° (c = 0,5 Methanol).

Analog wurden erhalten :

26a. D-1-Amino-indan-1-yl-carbonyl-L-leucyl-glycinamid (21 %), $[\alpha]_D^{20}$ = — 4° (c = 0,5 Methanol) ;
26b. L-1-Amino-indan-1-yl-carbonyl-L-leucyl-glycinamid (18 %), Fp = 82 bis 87 °C (Essigsäureethylester/Petrolether, $[\alpha]_D^{20}$ = — 27° (c = 0,5 Methanol).

## Beispiel 27

1-Amino-cyclohex-1-yl-carbonyl-L-leucyl-glycinamid

Zu 7,9 g 1-Benzyloxycarbonylamino-cyclohexan-1-carbonsäure und 4 ml Triethylamin in 75 ml Tetrahydrofuran wurden bei —10 °C unter Rühren 4 ml Chlorameisensäureisobutylester zugetropft. Nach 15 min wurden zu der erhaltenen Lösung des asymmetrischen Säureanhydrids 5,6 L-Leucyl-glycinamid in 50 ml Dimethylformamid zugegeben. Man rührte 1 h bei —10 °C, dann 16 h Raumtemperatur und dampfte anschließend im Vakuum ein. Der Rückstand wurde in Essigsäureethylester aufgenommen und nacheinander mit 10 % wäßriger Zitronensäure-Lösung, Soda-Lösung und Wasser gewaschen, über Natriumsulfat getrocknet und im Vakuum eingedampft. Man erhielt 7,5 g 1-Benzyloxycarbonylamino-cyclohex-1-yl-carbonyl-L-leucyl-glycinamid.

5,7 g der Benzyloxycarbonyl-Verbindung wurden in Methanol aufgenommen und über Palladium auf Kohle (10 %) hydriert. Man erhielt 3,6 g (93 %) 1-Amino-cyclohex-1-yl-carbonyl-L-leucyl-glycinamid, Fp = 136 bis 147 °C (Isopropanol/Ether/Hexan).

Analog Beispiel 27 wurden erhalten :

28. 1-Amino-cyclohep-1-yl-carbonyl-L-leucyl-glycinamid (49 %), Fp = 65 bis 70 °C (Aceton/Ether/Hexan), $[\alpha]_D^{20}$ = —8° (c = 0,8 Methanol) ;

29. 2-Amino-indan-2-yl-carbonyl-L-leucyl-glycinamid (61 %), Fp = 72 bis 80 °C (Essigsäureethylester/Pentan), $[\alpha]_D^{20}$ = —4° (c = 0,5 Methanol).

## Beispiel 30

1-Amino-cycloprop-1-yl-carbonyl-L-leucyl-glycinamid

Zu 2,3 g L-Leucyl-glyinamid in 30 ml Dioxan wurden bei 10 °C 1,7 ml Triethylamin und 3,6 g des N-Hydroxysuccinimidesters von 1-Butyloxycarbonylamino-cyclopropan-1-carbonsäure zugefügt. Das Reaktionsgemisch wurde 1 h bei 10 °C gerührt, anschließend wurden 10 ml Wasser zugegeben und die Reaktion 20 h bei Raumtemperatur fortgesetzt. Zur Aufarbeitung wurde in Essigsäureethylester aufgenommen und nacheinander mit 10 %iger wäßriger Zitronensäure-Lösung, Soda-Lösung und Wasser gewaschen, über Natriumsulfat getrocknet und im Vakuum eingedampft. Der Rückstand wurde durch Chromatographie an einer Kieselgelsäule (Elution mit Dichlormethan/Methanol 10 : 1 gereinigt. Man erhielt 3,1 g 1-Butyloxycarbonylamino-cycloprop-1-yl-carbonyl-L-leucyl-glyinamid.

Dieses wurde 15 min bei Raumtemperatur mit 30 ml einer ca. 6 normalen Lösung von Chlorwasserstoff in Dioxan behandelt. Anschließend wurde im Vakuum eingedampft, der Rückstand mehrmals mit Toluol zusammen evaporiert und aus Isopropanol/Ether kristallisiert. Ausbeute : 2,2 g (60 %) 1-Amino-cycloprop-1-yl-carbonyl-L-leucyl-glycinamid-Hydrochlorid, Fp = 110 bis 120 °C (Isopropanol/Ether).

## Beispiel 31

D-1,4-Thiazan-3-yl-carbonyl-L-leucyl-glycinamid und L-1,4-Thiazan-3-yl-carbonyl-L-leucyl-glycinamid

Zu 5,6 g N-Benzyloxycarbonyl-D,L-1,4-thiazan-3-carbonsäure in 40 ml Dioxan wurden bei 0 bis 10 °C 2,4 g N-Hydroxysuccinimid und 4,2 g Dicyclohexylcarbodiimid zugegeben. Nach 10 min wurde mit 3,8 g L-Leucyl-glycinamid versetzt und anschließend 16 h bei Raumtemperatur gerührt. Zur Aufarbeitung wurde in Essigsäureethylester aufgenommen und nacheinander mit 10 %iger Zitronensäure-Lösung, Soda-Lösung und Wasser gewaschen, über Natriumsulfat getrocknet und anschließend im Vakuum eingedampft. Der Rückstand wurde durch Chromatographie an einer Kieselgelsäure (Elution mit Dichlormethan/Methanol 15 : 1) chromatographiert. Man erhielt 4,6 g.N-Benzyloxycarbonyl-D,L-1,4-thiazan-3-yl-carbonyl-L-leucyl-glycinamid.

4,0 g des Benzyloxycarbonylderivats wurden mit 50 ml einer auf 0 °C gekühlten 40 %igen Lösung von Bromwasserstoff in Eisessig versetzt und 2 h bei 0 °C gerührt. Nach Eindampfen im Vakuum und mehrfachem evaporieren zusammen mit Toluol wurde der Rückstand in Wasser gelöst und mit einem stark basischen Ionenaustauscher auf pH 9 bis 10 gebracht. Nach Filtration vom Ionenaustauscher wurde eingedampft und an einer Kieselgelsäule chromatographiert. Elution mit Dichlormethan/Methanol 6 : 1 gab zunächst 0,9 g L-1,4-Thiazan-3-yl-carbonyl-L-leucyl-glycinamid (32 %), Fp = 90 bis 100 °C, $[\alpha]_D^{20}$ = —22° (c = 0,5 Methanol).

Weitere Elution mit demselben Lösungsmittel gab 1,1 g D-1,4-Thiazan-3-yl-carbonyl-L-leucyl-glycinamid (39 %), Fp = 152 bis 157 °C (Isopropanol/Ether/Hexan), $[\alpha]_D^{20}$ = —11° (c = 0,5 Methanol).

## Beispiel 32

Pyrrol-2-yl-carbonyl-L-leucyl-glycindimethylamid

2,0 g Pyrrol-2-yl-carbonyl-L-leucyl-glycinethylester in 35 ml Methanol wurden mit 20 ml 40 % wäßriger Dimethylamin-Lösung versetzt. Nach 48 h wurde eingedampft und der Rückstand an einer Kieselgelsäule (Elution mit Dichlormethan/Methanol 20 : 1) chromatographiert. Man erhielt 1,3 g (65 %) Pyrrol-2-yl-carbonyl-L-leucyl-glycindimethylamid, Fp = 133 bis 137°C (Dichlormethan/Ether/Hexan), $[\alpha]_D^{20}$ = — 13° (c = 0,5 Methanol).

Analog Beispiel 32 wurden erhalten :

33. Indol-2-yl-carbonyl-L-leucyl-glycindimethylamid (479 %), Fp = 150 bis 155 °C (Dichlormethan/Ether/Hexan), $[\alpha]_D^{20}$ = +8° (c = 0,5 Methanol).

34. Pyrrol-2-yl-carbonyl-L-leucyl-glycinmethylamid (70 %), Fp = 95 bis 108 °C (Dichlormethan/Ether/-Hexan), $[\alpha]_D^{20}$ = + 4° (c = 0,5 Methanol).

35. Indol-2-yl-carbonyl-L-leucyl-glycinmethylamid (87 %), Fp = 108 bis 114 °C (Dichlormethan/Ether/Hexan), $[\alpha]_D^{20}$ = + 9° (c = 0,5 Methanol).

Beispiele für galenische Zubereitungen :

Beispiel A

Auf einer Tablettenpresse wurden in üblicher Weise Tabletten folgender Zusammensetzung gepreßt :

| | | |
|---|---|---|
| 40 | mg | Substanz des Beispiels 9 |
| 120 | mg | Maisstärke |
| 13,5 | mg | Gelatine |
| 45 | mg | Milchzucker |
| 2,25 | mg | Aerosil[R] (chemisch reine Kieselsäure in submikroskopisch feiner Verteilung) |
| 6,75 | mg | Kartoffelstärke (als 6 %iger Kleister) |

Beispiel B

In üblicher Weise wurden Dragees folgender Zusammensetzung hergestellt :

| | |
|---|---|
| 20 mg | Substanz des Beispiels 9 |
| 60 mg | Kernmasse |
| 60 mg | Verzuckerungsmasse |

Die Kernmasse besteht aus 9 Teilen Maisstärke, 3 Teilen Milchzucker und 1 Teil Luviskol[R] VA 64 (Vinylpyrrolidon-Vinylacetat-Mischpolymerisat 60 : 40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees wurden anschließend mit einem magensaftresistenten Überzug versehen.

**Patentansprüche** (für die Vertragsstaaten : BE, CH, DE, FR, GB, IT, LI, NL, SE)

1. N-Acylderivate von Dipeptiden der Formel I

$$X—CO—L—Leu—NH—CH_2—CO—R \quad I,$$

in der

X einen gesättigten cycloaliphatischen Kohlenwasserstoffrest mit 3 bis 10 Kohlenstoffatomen oder einen Indanyl- oder 1,2,3,4-Tetrahydronaphthalinrest bedeutet, in welchen Resten das C-Atom, welches mit der Carbonylgruppe verbunden ist, noch eine Aminogruppe tragen kann, einen L-Homopyrrolidinylrest oder ein über ein C-Atom gebundenes gesättigtes oder ungesättigtes 4- bis 6-gliedriges heterocyclisches Ringsystem mit bis zu 2 Heteroatomen darstellt, wobei der Heterocyclus auch mit einem Benzolkern kondensiert sein kann und wobei — falls der Heterocyclus Stickstoff enthält — dieser auch eine Oxo- oder eine $C_1$-$C_3$-Alkylgruppe enthalten kann, und

R eine $C_1$-$C_5$-Alkoxygruppe oder der Rest $NR^1R^2$ ist, worin $R^1$ und $R^2$ gleich oder verschieden sind und Wasserstoffatome oder $C_1$-$C_5$-Alkylgruppen bedeuten,

wobei jedoch X

a) kein Pyrrolidin-2-ylrest oder 5-Oxo-pyrrolidin-2-ylrest
b) kein Cyclopentylrest, wenn R eine Amino- oder Ethylaminogruppe bedeutet, und
c) kein 4-Thiazolidinrest, wenn R eine Aminogruppe bedeutet, sein kann,

sowie deren Salze mit physiologisch verträglichen Säuren.

2. Verfahren zur Herstellung der N-Acylderivate von Dipeptiden der Formel I gemäß Anspruch 1, dadurch gekennzeichnet, daß man die Verbindungen X—COOH, L-Leu und $H_2N—CH_2—CO—R$, worin X und R die angegebene Bedeutung haben, in dieser Reihenfolge nach den in der Peptidchemie üblichen Methoden kondensiert und die so erhaltenen Verbindungen gegebenenfalls in ihre Salze mit physiologisch verträglichen Säuren überführt.

3. Verfahren gemäß Anspruch 2, dadurch gekennzeichnet, daß man

a) falls X keine basische Aminogruppe enthält — eine Carbonsäure der Formel II

X—COOH II,

auf ein Dipeptid der Formel III

$L—Leu—CO—NH—CH_2—CO—R$ III,

worin R die angegebene Bedeutung besitzt, einwirken läßt oder

b) falls X eine basische Aminogruppe enthält — die an der Aminogruppe durch eine Schutzgruppe geschützte Verbindung der Formel II mit dem Dipeptid der Formel III kondensiert und anschließend die Schutzgruppe abspaltet

und die so erhaltenen Verbindungen gegebenenfalls in ihre Salze mit physiologisch verträglichen Substanzen überführt.

4. Arzneimittel enthaltend eine Verbindung der Formel I gemäß Anspruch 1.

5. Verbindung der Formel I gemäß Anspruch 1 zur Verwendung bei der Therapie von Krankheiten.

6. Verbindungen der Formel I gemäß Anspruch 1 zur Verwendung bei der Bekämpfung von Morbus Parkinson, parkinsonoider Zustände und Depressionen.

**Patentanspruch** (für den Vertragsstaat AT)

Verfahren zur Herstellung von N-Acylderivate von Dipeptiden der Formel I

$X—CO—L—Leu—NH—CH_2—CO—R$ I,

in der

X einen gesättigten cycloaliphatischen Kohlenwasserstoffrest mit 3 bis 10 Kohlenstoffatomen oder einen Indanyl- oder 1,2,3,4-Tetrahydronaphthalinrest bedeutet, in welchen Resten das C-Atom, welches mit der Carbonylgruppe verbunden ist, noch eine Aminogruppe tragen kann, einen L-Homopyrrolidinylrest oder ein über ein C-Atom gebundenes gesättigtes oder ungesättigtes 4-bis 6-gliedriges heterocyclisches Ringsystem mit bis zu 2 Heteroatomen darstellt, wobei der Heterocyclus auch mit einem Benzolkern kondensiert sein kann und wobei — falls der Heterocyclus Stickstoff enthält — dieser auch eine Oxo- oder eine $C_1$-$C_3$-Alkylgruppe enthalten kann, und

R eine $C_1$-$C_5$-Alkoxygruppe oder der Rest $NR^1R^2$ ist, worin $R^1$ und $R^2$ gleich oder verschieden sind und Wasserstoffatome oder $C_1$-$C_5$-Alkylgruppen bedeuten,

wobei jedoch X

a) kein Pyrrolidin-2-ylrest oder 5-Oxo-pyrrolidin-2-ylrest
b) kein Cyclopentylrest, wenn R eine Amino- oder Ethylaminogruppe bedeutet, und
c) kein 4-Thiazolidinrest, wenn R eine Aminogruppe bedeutet,

sein kann,

sowie deren Salze mit physiologisch verträglichen Säuren, dadurch gekennzeichnet, daß man die Verbindungen X—COOH, L-Leu und $H_2N—CH_2—CO—R$, worin X und R die angegebene Bedeutung haben, in dieser Reihenfolge nach den in der Peptidchemie üblichen Methoden kondensiert und die so erhaltenen Verbindungen gegebenenfalls in ihre Salze mit physiologisch verträglichen Säuren überführt.

**Claims** (for the Contracting States : BE, CH, DE, FR, GB, IT, LI, NL, SE)

1. An N-acyl derivative of a dipeptide of the formula I

$X—CO—L—Leu—NH—CH_2—CO—R$ I,

where

X is a saturated cycloaliphatic hydrocarbon radical of 3 to 10 carbon atoms, indanyl or 1,2,3,4-tetrahydronaphthyl, in which radicals the carbon atom which is bonded to the carbonyl group can also carry an amino group, or X is L-homopyrrolidinyl or a saturated or unsaturated 4-membered, 5-membered or 6-membered heterocyclic ring system bonded via a carbon atom and containing up to 2 heteroatoms, and the heterocyclic structure may furthermore be fused to a benzene nucleus, and, if the heterocyclic structure contains nitrogen, it may also contain an oxo or a $C_1$-$C_3$-alkyl group, and

R is $C_1$-$C_5$-alkoxy or a radical $NR^1R^2$, where $R^1$ and $R^2$ are identical or different and are each hydrogen or $C_1$-$C_5$-alkyl,
but where X

a) cannot be pyrrolidin-2-yl or 5-oxopyrrolidin-2-yl,
b) cannot be cyclopentyl if R is amino or ethylamino, and
c) cannot be 4-thiazolidinyl if R is amino,

and its salts with physiologically tolerated acids.

2. A process for the preparation of an N-acyl derivative of a dipeptide of the formula I as claimed in claim 1, wherein the compounds X—COOH, L-Leu and $H_2N—CH_2—CO—R$, where X and R have the specified meanings, are condensed in this order by a method usually used in peptide chemistry, and, if desired, the compound thus obtained is converted into its salts with physiologically tolerated acids.

3. A process as claimed in claim 2, wherein

a) if X does not contain a basic amino group — a carboxylic acid of the formula II

$$X—COOH \qquad II$$

is allowed to act on a dipeptide of the formula III

$$L—Leu—CO—NH—CH_2—CO—R \qquad III,$$

where R has the specified meanings, or

b) if X contains a basic amino group — the compound of the formula II, which is protected by a protective group on the amino group, is condensed with a dipeptide of the formula III, and the protective group is then split off.

4. A drug containing a compound of the formula I as claimed in claim 1.

5. A compound of the formula I as claimed in claim 1 for use in the therapy of disorders.

6. A compound of the formula I as claimed in claim 1 for use in the treatment of Parkinson's disease, Parkinson-like conditions and depressions.

**Claim** (for the Contracting State AT)

A process for the preparation of an N-acyl derivative of a dipeptide of the formula I

$$X—CO—L—Leu—NH—CH_2—CO—R \qquad I,$$

where

X is a saturated cycloaliphatic hydrocarbon radical of 3 to 10 carbon atoms, indanyl or 1,2,3,4-tetrahydronaphthyl, in which radicals the carbon atom which is bonded to the carbonyl group can also carry an amino group, or X is L-homopyrrolidinyl or a saturated or unsaturated 4-membered, 5-membered or 6-membered heterocyclic ring system bonded via a carbon atom and containing up to 2 heteroatoms, and the heterocyclic structure may furthemore be fused to a benzene nucleus, and, if the heterocyclic structure contains nitrogen, it may also contain an oxo or a $C_1$-$C_3$-alkyl group, and

R is $C_1$-$C_5$-alkoxy or a radical $NR^1R^2$, where $R^1$ and $R^2$ are identical or different and are each hydrogen or $C_1$-$C_5$-alkyl,
but where X

a) cannot be pyrrolidin-2-yl or 5-oxopyrrolidin-2-yl,
b) cannot be cyclopentyl if R is amino or ethylamino, and
c) cannot be 4-thiazolidinyl if R is amino,

and its salts with physiologically tolerated acids, wherein the compounds X—COOH, L-Leu and $H_2N—CH_2—CO—R$, where X and R have the specified meanings, are condensed in this order by a method usually used in peptide chemistry, and, if desired, the compound thus obtained is converted into its salts with physiologically tolerated acids.

**Revendications** (pour les Etats contractants : BE, CH, DE, FR, GB, IT, LI, NL, SE)

1. Dérivés N-acylés de dipeptides de la formule I

$$X{-}CO{-}L{-}Leu{-}NH{-}CH_2{-}CO{-}R \qquad I,$$

dans laquelle

X représente un radical hydrocarboné cycloaliphatique saturé comportant de 3 à 10 atomes de carbone, ou un radical indanyl- ou 1,2,3,4-tétrahydronaphtalène, radicaux dans lesquels l'atome de carbone qui est lié au radical carbonyle peut encore porter un groupe amino, un radical L-homopyrrolidinyle, ou un système cyclique à hétérocycle possédant jusqu'à 2 hétéroatomes, comportant 4 à 6 chaînons, saturé ou insaturé, lié par l'intermédiaire d'un atome de carbone, où l'hétérocycle peut également être condensé avec un noyau benzénique et où — au cas où l'hétérocycle contient de l'azote — celui-ci peut également contenir un radical oxo ou un radical alkyle en $C_1$-$C_3$ et

R représente un radical alcoxy en $C_1$-$C_5$ ou le groupe $NR^1R^2$ où $R^1$ et $R^2$ sont identiques ou différents et représentent des atomes d'hydrogène ou des radicaux alkyle en $C_1$-$C_5$,
mais avec cependant la particularité que X

a) ne représente pas de radical pyrrolidine-2-yle ou de radical 5-oxo-pyrrolidine-2-yle,
b) ne représente pas de radical cyclopentyle lorsque R représente un groupe amino ou éthylamino et
c) ne représente pas de radical 4-thiazolidinyle lorsque R représente un groupe amino,

comme aussi les sels de ces composés avec des acides physiologiquement compatibles.

2. Procédé de préparation de dérivés N-acylés de dipeptides de la formule I suivant la revendication 1, caractérisé en ce que l'on condense les composés X—COOH, L-Leu et $H_2N{-}CH_2{-}CO{-}R$, où X et R possèdent les significations qui leur ont été précédemment attribuées, dans la suite indiquée, selon des procédés usuels de la chimie des peptides et on transforme éventuellement les composés ainsi obtenus en leurs sels avec des acides physiologiquement compatibles.

3. Procédé suivant la revendication 2, caractérisé en ce que

a) au cas où X contient un radical amino basique — on fait réagir un acide carboxylique

$$X{-}COOH \qquad II,$$

sur un dipeptide de la formule III

$$L{-}Leu{-}CO{-}NH{-}CH_2{-}CO{-}R \qquad III,$$

dans laquelle R possède les significations qui lui ont été précédemment attribuées, ou

b) au cas où X contient un radical amino basique — on condense le composé de la formule II protégé par un radical protecteur sur le groupe amino, avec le dipeptide de la formule III et on sépare ensuite le radical protecteur

et on transforme éventuellement les composés ainsi obtenus en leurs sels avec des substances physiologiquement compatibles.

4. Médicament contenant un composé de la formule I suivant la revendication 1.

5. Composés de la formule 1 suivant la revendication 1 en vue de leur emploi au cours du traitement thérapeutique de maladies.

6. Composés de la formule 1 suivant la revendication 1 en vue de leur emploi pour la lutte contre la maladie de Parkinson, des états parkinsoniens et des dépressions.

**Revendication** (pour l'Etat contractant AT)

Procédé de préparation de dérivés N-acylés de dipeptides de la formule I

$$X{-}CO{-}L{-}Leu{-}NH{-}CH_2{-}CO{-}R \qquad I,$$

dans laquelle

X représente un radical hydrocarboné cycloaliphatique saturé comportant de 3 à 10 atomes de carbone, ou un radical indanyl- ou 1,2,3,4-tétrahydronaphtalène, radicaux dans lesquels l'atome de carbone qui est lié au radical carbonyle peut encore porter un groupe amino, un radical L-homopyrrolidinyle, ou un système cyclique à hétérocycle possédant jusqu'à 2 hétéroatomes, comportant 4 à 6 chaînons, saturé ou insaturé, lié par l'intermédiaire d'un atome de carbone, où l'hétérocycle peut

également être condensé avec un noyau benzénique et où — au cas où l'hétérocycle contient de l'azote — celui-ci peut également contenir un radical oxo ou un radical alkyle en $C_1$-$C_3$ et

R représente un radical alcoxy en $C_1$-$C_5$ ou le groupe $NR^1R^2$ où $R^1$ et $R^2$ sont identiques ou différents et représentent des atomes d'hydrogène ou des radicaux alkyle en $C_1$-$C_5$, mais avec cependant la particularité que X

a) ne représente pas de radical pyrrolidine-2-yle ou de radical 5-oxo-pyrrolidine-2-yle,
b) ne représente pas de radical cyclopentyle lorsque R représente un groupe amino ou éthylamino et
c) ne représente pas de radical 4-thiazolidinyle lorsque R représente un groupe amino,

comme aussi de leurs sels avec des acides physiologiquement compatibles, caractérisé en ce que l'on condense les composés X—COOH, L-Leu et $H_2N$—$CH_2$—CO—R, où X et R possèdent les significations qui leur ont été précédemment attribuées, dans la suite indiquée, selon des procédés usuels de la chimie des peptides et on transforme éventuellement les composés ainsi obtenus en leurs sels avec des acides physiologiquement compatibles.